# NEUE EUROPÄISCHE PATENTSCHRIFT

(11) **EP 2 032 065 B2**
(45) Veröffentlichungstag und Bekanntmachung des Hinweises auf die Entscheidung über den Einspruch: **06.12.2017**
(45) Hinweis auf die Patenterteilung: 29.12.2010
(21) Anmeldenummer: 06754687.9
(22) Anmeldetag: 06.07.2006
(51) Int. Cl.: A61B 18/22

(54) **VORRICHTUNG FÜR DIE LASERCHIRURGIE**
DEVICE FOR LASER SURGERY
DISPOSITIF DE CHIRURGIE AU LASER

(30) Priorität: 26.06.2006 US 426415
(43) Veröffentlichungstag der Anmeldung: 11.03.2009
(60) Teilanmeldung: 10187013.7
(73) Patentinhaber: Rolle + Rolle GmbH + Co. KG, 5020 Salzburg (AT)
(72) Erfinder: ROLLE, Ingeborg, 82291 Mammendorf (DE); KOETH, Johannes, Bernhard, 97218 Gerbrunn (DE)
(74) Vertreter: Grünecker Patent- und Rechtsanwälte PartG mbB
(86) Internationale Anmeldenummer: PCT/EP2006/006632
(87) Internationale Veröffentlichungsnummer: WO 2008/000294

(56) Entgegenhaltungen:
- WO-A-2005/004737
- WO-A1-03/049633
- WO-A2-01/74230
- US-A- 5 330 517
- US-A- 5 910 140
- US-A1- 2003 216 717
- " Technik der Lasersegmentresektion mit dem 1318 nm Nd:YAG Laser"
- Ceralas HPD 120 User Manual
- LIVSBITS D.A. ET AL: '8W continuous wave operation of InGaAsN lasers at 1.3pm' ELECTRONICS LETTERS Bd. 36, Nr. 16, 03 August 2000, Seiten 1381 - 1382
- QU Y. ET AL: 'High-power 1.3-pm InGaAsN strain-compensated lasers fabricated with pulsed anodic oxidation' APPLIED PHYSICS LETTERS Bd. 85, Nr. 22, 29 November 2004, Seiten 5149 - 5151

## Beschreibung

Die Erfindung betrifft eine Vorrichtung für die Laserchirurgie mit einem Laser und Mitteln zum Einkoppeln von Laserlicht des Lasers in eine Lichtleitfaser.

Derartige Vorrichtungen für die Laserchirurgie sind beispielsweise aus der DE 91 162 16 U bekannt. Auch die DE 44 08 746 C2 zeigt einen Laserkatheter zur Bypasschirurgie. Hier werden zum Beispiel Nd: YAG-Laser erwähnt. Diese Laser weisen eine Laserlinie bei 1064 nm auf. Aus der Druckschrift US 5 910140 its eine medizinische laservorrichtung mit einem Nd:YACO₃ laserkristall bekannt.

Aus der Druckschrift WO0174230 ist ein Halbleiterlaser zur Tumor therapie bekannt.

Aufgabe der vorliegenden Erfindung ist es, eine Vorrichtung für die Laserchirurgie zu verbessern. Diese Aufgabe wird gelöst durch eine Vorrichtung nach Anspruch 1.

Für die Laserchirurgie von Geweben im Allgemeinen, insbesondere aber für die Chirurgie von Lungengewebe, hat sich herausgestellt, dass eine Wellenlänge zwischen 1100 nm und 1400 nm vorteilhaft ist. In diesem Wellenlängenbereich stellt sich eine Absorption der Laserstrahlung in wasserhaltigem Gewebe also auch Lungengewebe ein, die es erlaubt zügig das Gewebe zu durchtrennen oder zu schneiden und gleichzeitig eine ausgedehnte Koagulation an stark durchblutetem Gewebe zu erzeugen. Beim Lungengewebe wird ebenfalls gleichzeitig ein weiterer sehr wichtiger Effekt nämlich die Verschweißung von Luftfisteln erreicht. In diesem Wellenlängenbereich sind zwar Lasersysteme verfügbar, jedoch sind diese in der Regel sehr unhandlich, da beispielsweise Gaslaser in der Regel eine aufwendige Wasserkühlung benötigen, sodass ein Gerät dieser Art sehr schwer ist oder die verfügbaren Ausgangsleistungen zu gering sind.

Für die Laserchirurgie im oben genannten Wellenlängenbereich ist ein Laser mit einer Ausgangsleistung von mindestens 25 W wünschenswert.

Auf Grund kürzlicher materialtechnischer Entwicklungen ist es möglich, Festkörperlaser wie etwa Halbleiterlaser und/oder Faserlaser mit derartigen Ausgangsleistungen, wie sie für die Laserchirurgie, insbesondere für die Lungenlaserchirurgie, benötigt werden, zur Verfügung zu stellen und dabei eine Wellenlänge im Bereich von 1100 bis 1400 nm zu erzeugen.

Besonders bevorzugt ist ein Wellenlängenbereich oberhalb von 1250 1275, 1300, 1308 oder 1310 nm. In diesem Wellenlängenbereich zeigt die Absorption von Wasser eine nicht allzu große Abhängigkeit von der Wellenlänge, sodass bestimmte Wellenlängenschwankungen in diesem Bereich nicht automatisch zu einem anderen chirurgischen Verhalten der Laserstrahlung führen. Andererseits sollte die Laserstrahlung auch unterhalb von einer Wellenlänge von 1325, 1300, 1275, 1250, 1225 oder 1200 nm liegen, da ansonsten der Plateaubereich verlassen wird. Auf diesem Plateaubereich stellt sich eine relativ günstige Absorption und ein vorteilhaftes Verhältnis von Absorption zu Streuung in Geweben mit relativ hohem Wassergehalt und somit auch in Lungengewebe ein.

Vorteilhaft sind weiterhin Laser mit einem Laserlicht, das eine gewisse spektrale Halbwertsbreite aufweist. Hier wird die spektrale Breite des Spektrums bei der Hälfte der Maximalintensität bestimmt. Die Halbwertsbreite beträgt z. B. mindestens 2, 3, 4, 5, 7, 10, 12 oder 15 nm. Die Obergrenze für die Halbwertsbreite kann z. B. 2, 3, 4, 5, 7, 10, 12, 15, 20, 25, 30 oder 40 nm sein. Durch die Abdeckung eines größeren Spektralbereichs wird die Vorrichtung gegen geringe Wellenlängenschwankungen unempfindlicher, sodass ein konstantes Chirurgieverhalten des Laserlichts während einer Operation gewährleistet wird. Andererseits soll das Spektrum auch nicht zu breit sein, damit keine Spektralanteile mit einer zu hohen Absorption in dem Gewebe vorliegen.

Die Ausgangsleistung des Lasers ist größer als 50 W. Hier ist beispielsweise eine Leistung von mindestens 80 W bevorzugt.

Der Laser kann sowohl ein Dauerstrich als auch ein gepulster Laser sein.

Vorteilhaft ist weiterhin ein Laser, der mehrere Laserelemente umfasst. Dies können mindestens oder genau 15, 19, 20, 25, 30, 35, 38, 40, 50, 60, 70, 80, 90, 100, 150 Laserelemente sein. Die einzelnen Laserelemente können so mit einer geringeren Leistung als mit der gewünschten Gesamtausgangsleistung betrieben werden, was die Lebensdauer dieser Laserelemente erhöht bzw. größere Ausgangsleistungen erst ermöglicht.

Weiterhin sind hier vorteilhafterweise Mittel vorgesehen, mit denen die Laserstrahlen dieser einzelnen Laserelemente in eine gemeinsame Lichtleitfaser eingekoppelt werden können. An sich ist es auch denkbar, für jedes Laserelement eine einzelne Lichtleitfaser vorzusehen. Diese einzelnen Fasern können dann gebündelt werden und das Licht derselben erst am Ende der Lichtleitfaser mit einer Optik zusammengeführt werden. Derartige Mittel zum Einkoppeln von Laserlicht können eine oder mehrere Linsen und/oder Spiegel umfassen, wobei hier auch zylindrische Linsen/Linsenarrays und/oder Spiegel vorgesehen sein können.

Die einzelnen Laserelemente und/oder Laser sind oder umfassen Halbleiterlaser. Nicht der Erfindung entsprechend können beispielsweise einzelne Faserlaser gebündelt angeordnet werden, um das Licht in eine gemeinsame Ausgangslichtleitfaser zu geben. Hier können aber auch ein oder mehrere Faserkoppler vorgesehen sein, mit denen das Licht aus zwei oder mehr Fasern in eine einzelne Faser eingekoppelt wird.

Auch mit Halbleiterlasern kann im gewünschten Wellenlängenbereich die benötigte Ausgangsleistung erreicht werden. Hierzu wird in der Regel über eine Strahloptik das Licht von mehreren Laserelementen in eine Lichtleitfaser eingekoppelt.

Die Halbleiterlaser können entweder elektrisch oder optisch gepumpt werden. Ein optisches Pumpen kann z.B. mit anderen Halbleiterlasern mit einer Wellenlänge kürzer als 1100 nm erfolgen. Die Faserlaser werden in der Regel optisch gepumpt wie etwa mit Halbleiterlaserdioden, wie sie in diesem Dokument beschrieben werden. In der Regel werden die Pumplaser eine Wellenlänge von unterhalb 1100 nm haben, jedoch zumindest unterhalb der Wellenlänge des Faserlasers.

Vorteilhafterweise ist der Laser ein Quantenpunktlaser bzw. ein Quantenfilmlaser. Mit derartigen Laserelementen ist es mit einer vertretbaren Anzahl von Laserelementen möglich in dem gewünschten Wellenlängenbereich die benötigte Ausgangsleistung zur Verfügung zu stellen. Derartige Quantenpunktlaser können auf dem GaAs-AlGaAs-Materialsystem beruhen. Die Quantenpunkte des Quantenpunktlasers können hierbei in Quantenfilmen (auch Quantentröge genannt) vorgesehen sein, sodass das Confinement der Ladungsträger im Bereich der Quantenpunkte durch das Confinement der Ladungsträger in dem Quantenfilm verbessert wird.

Quantenpunkte können jedoch auch ohne Quantenfilme bzw. außerhalb von eventuellen Quantenfilmen vorgesehen sein.

Quantenpunkte können GaInAs, GaInAsN und/oder GalnSb umfassen oder daraus bestehen. Die Quantenpunkte haben eine Bandlücke, die geringer ist als diejenige des umgebenden Materials. Dadurch werden die Ladungsträger auf Energieniveaus, die u. a. durch die Größe des Quantenpunkts vorgegeben werden, gebracht, sodass hier auch Laserwellenlängen möglich werden, die nicht der Bandlücke des Quantenpunktmaterials entsprechen.

Der Wellenleiter des Halbleiterlasers bestimmt die Lichtführung innerhalb des Halbleitermaterials. Hier wird für den Laser für die Vorrichtung für die Laserchirurgie ein relativ breiter Wellenleiter bevorzugt, da das Licht sich somit über einen großen Bereich in dem Halbleiterlasermaterial erstreckt und die Inhomogenitäten durch die Ausbildung von Quantenpunkten, Quantenfilmen oder sonstigen Grenzflächen hohe Leistungen nicht behindern, beispielsweise durch Streuung an Grenzflächen oder Grenzflächendefekten oder an den Quantenpunkten.

Der Wellenleiter hat eine Breite von mindestens 200, 250, 300, 400, 500 oder 600 nm. Der Wellenleiter wird durch eine Brechungsindexdifferenz zwischen dem Wellenleiterinnerem und dem Wellenleiteräußeren definiert. Der Wellenleiter ist bevorzugterweise so ausgebildet, dass sich nur eine transversale Lichtmode einstellt. Es können aber auch zwei oder drei transversale Moden gegeben sein, da so höhere Leistungen möglich werden, ohne jedoch ein wohldefiniertes Strahlprofil zu verlieren.

Der Wellenleiter kann GaₓIn₍₁₋ₓ₎AsᵤPᵥ-N_{w}Sb_{(1-u-v-w)} umfassen oder daraus bestehen, wobei x, u, v und w die Werte 0 bis 1 und alle Zwischenwerte oder Werte aus allen möglichen Zwischenintervallen annehmen kann und u+v+w kleiner oder gleich 1 ist.

Der Halbleiterlaser kann auch ein Quantenfilmlaser sein, mit einem Quantenfilm der GaᵤIn₁₋ᵤ AsₓN_{y}P_{1-x-y} umfasst oder daraus besteht, wobei u, x und y die Werte 0 bis 1 oder alle Zwischenwerte oder Werte aus allen möglichen Zwischenintervallen annehmen kann solange x+y≤1 ist. Mit derartigen Materialien sind gewünschte Ausgangsleistungen in dem vorgegebenen Wellenlängenbereich möglich. Dies erfolgt unter Zusammenfassung von einer relativ großen Anzahl von Laserelementen, damit die benötigte insgesamte Ausgangsleistung des Lasers erreichbar erscheint.

Nicht entsprechend der Erfindung sind auch Festkörperscheibenlaser für die Erzeugung von Hochleistungslasem in dem benötigten Wellenlängenbereich vorteilhaft möglich. Bei diesen Lasern hat ein Kristall eine Scheibenform und ist auf einer Seite verspiegelt. Das Laserlicht tritt auf der gegenüberliegenden Seite aus. Solche Scheiben können sehr gut gekühlt werden, so dass hohe Leistungen möglich werden.

In jedem Fall sind zylindrische Linsen und/oder Linsenarrays und/oder Spiegel für die Einkopplung des Lichts von mehreren Laserelementen vorteilhaft.

Die Vorrichtung weist eine Kopplung auf, an die lösbar Lichtleitfasern angeschlossen werden können. Während einer Operation werden die Lichtleitfasern leicht verschmutzt, sodass diese auswechselbar sein sollten.

Die Lichtleitfaser weist vorteilhafterweise ein Griffteil auf, da dies die Manipulation der Lichtleitfaser vereinfacht. In dem Griffteil kann auch eine Optik vorgesehen sein, mit der das aus der Lichtleitfaser austretende Licht auf einen Fokus fokussiert wird. Zwingend notwendig ist dies jedoch nicht, da ein Bestrahlungsbereich von mehreren Millimetern Durchmesser auch ohne Optik erreichbar ist.

Die Optik ist vorzugsweise ebenfalls auswechselbar. Zum Einen wird die Optik auch leicht verschmutzt, zum Anderen können mit verschiedenen Optiken verschiedene Arbeitsabstände und verschiedene Fokusgrößen erreicht werden.

Die Vorrichtung weist weiterhin eine Luftkühlung zum Kühlen des Lasers bzw. der Vorrichtung auf. Eine Wasserkühlung ist hier nicht vorgesehen, da die Kühlluftkühlung bei einem Halbleiterlaser ausreichen sollte. Eine Wasserkühlung wird jedoch auch nicht ausgeschlossen.

Weiterhin kann die Vorrichtung eine Temperaturregeleinrichtung aufweisen, mit der die Temperatur des Halbleiterlasers eingestellt werden kann. Dies kann beispielsweise ein Peltierelement umfassen. Dies dient zum Einen dazu die Abwärme abzuführen. Andererseits kann mit der Temperatur auch die Wellenlänge des emittierten Lichts eingestellt werden. Das Peltierelement kann wasser- und/oder luftgekühlt sein. Auch ist lediglich eine Wasserkühlung ohne Peltierelement möglich. Die Wasserkühlung kann hierbei aber auch recht klein ausfallen.

Bevorzugte Ausführungsformen der Erfindung sollen anhand der beiliegenden Figuren erläutert werden. Dabei zeigt:
- Figur 1: eine Vorrichtung für die Laserchirurgie;
- Figur 2: eine schematische Schnittansicht des distalen Endes der Lichtleitfaser;
- Figur 3: die Anordnung von dem Halbleiterlaser, einer Einkoppeloptik und einem Fase- rende in schematischer dreidimensionaler Ansicht;
- Figur 4: schematische dreidimensionale Ansichten einer Laseranordnung;
- Figur 5: eine schematische Ansicht des Aufbaus eines Halbleiterquantenpunktlasers;
- Figur 6: ein beispielhaftes Spektrum des Lasers.

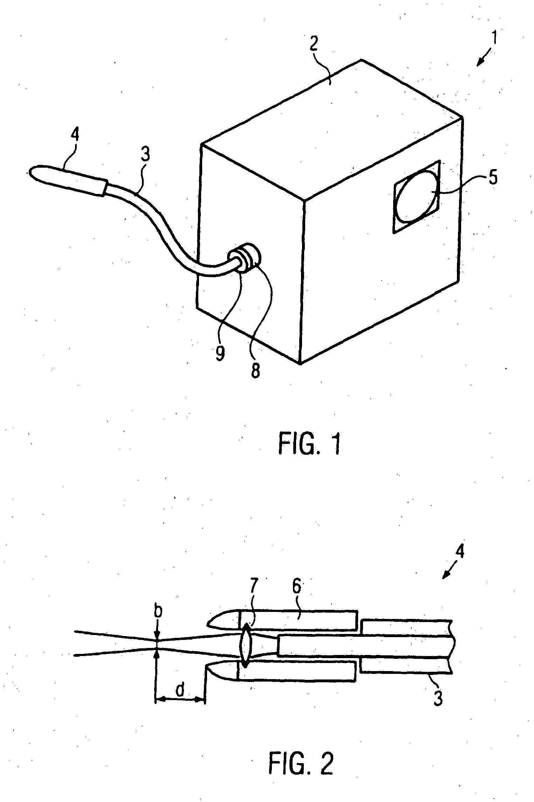

In Figur 1 ist eine Vorrichtung 1 für die Laserchirurgie gezeigt. Die Vorrichtung umfasst ein Gehäuse 2 mit einer Kopplung 8, in die eine Lichtleitfaser 3 mit einem Stecker 9 eingekoppelt werden kann. Die Lichtleitfaser 3 hat ein Ende mit einem Griffteil 4. Das Gehäuse 2 verfügt weiterhin über einen Lüfter 5 für die Luftkühlung.

Das Ende der Lichtleitfaser 3 mit dem Griffteil 4 ist in Figur 2 schematisch in einer Schnittansicht dargestellt. Die Lichtleitfaser 3 endet in einem abnehmbaren Optikhalter 6, in dem eine Optik 7, hierzu symbolisch durch eine einzelne Linse 7 dargestellt, angeordnet ist. Das aus der Lichtleitfaser 3 austretende Licht wird durch diese Optik gebündelt, sodass sich in einem Arbeitsabstand d ein Fokus mit einer Halbwertsbreite b ausbildet. Die Lichtleitfaser 3 hat einen lichtleitenden Faserkem sowie eine Ummantelung. Der bevorzugte Arbeitsabstand d beträgt einige Zentimeter. Besonders bevorzugt ist ein Arbeitsabstand von 1 bis 5 cm, wie etwa 1,5 cm (± 0,5 cm) oder 2,5 cm (± 1,0 cm) oder 3,5 cm (± 1,0 cm) oder 4,5 cm (± 0,5 cm). Die Halbwertsbreite b im Fokusbereich beträgt einige Millimeter. Der Strahldurchmesser im Fokus kann beispielsweise 0,5 mm sein.

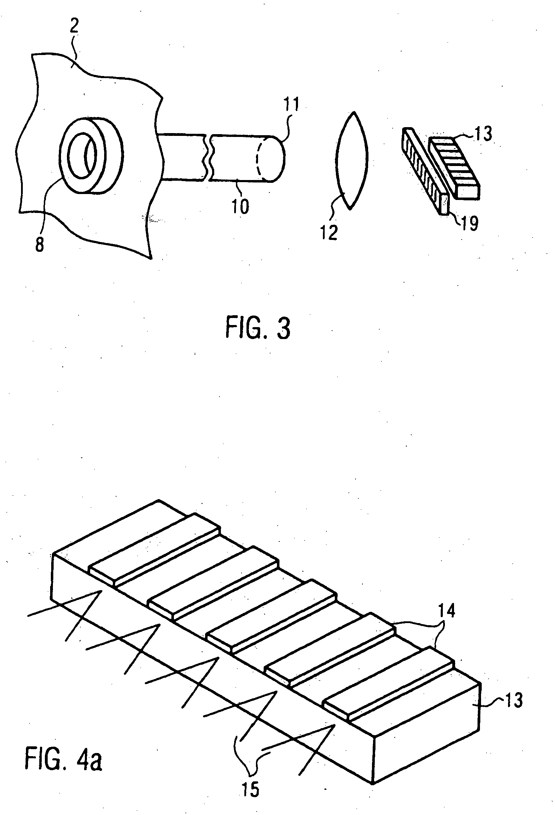

In Figur 3 ist ein Teil des Gehäuses 2 mit der Kopplung 8 dargestellt. Hier ist schematisch ein Faserende 11 einer internen Lichtleitfaser 10 dargestellt, in die das Licht des Lasers 13 eingekoppelt wird. Der Laser 13 ist hier als Laserbarren ausgeführt. Zwischen dem Laserbarren 13 und dem Ende 11 der Lichtleitfaser 10 ist eine Einkoppeloptik 12 vorgesehen, mit der das divergent aus dem Barren 13 austretende Licht auf das Lichtleitfaserende 11 gebündelt wird. Die Einkoppeloptik kann eine zylindrische Linse oder einen zylindrischen Spiegel umfassen. Bevorzugt ist beispielsweise auch ein Zylinderlinsenarray 19, wobei jedem Laserelement bevorzugt ein Zylinderinsenelement des Zytindeninsenarrays zugeordnet ist.

Der Barren 13 ist in Figur 4a schematisch dargestellt. In ihm sind verschiedene Halbleiterlaserelemente 14 angeordnet, wobei aus jedem Element 14 ein Lichtlaserstrahl 15, der sehr divergent sein kann, austritt.

In dem Barren 13 in Figur 4a sind Laserelemente 14 nebeneinander angeordnet. Ein solcher Laserbarren kann bis zu 20, 25, 30, 35, 40, 45 oder 50 Laserelemente 14 umfassen.

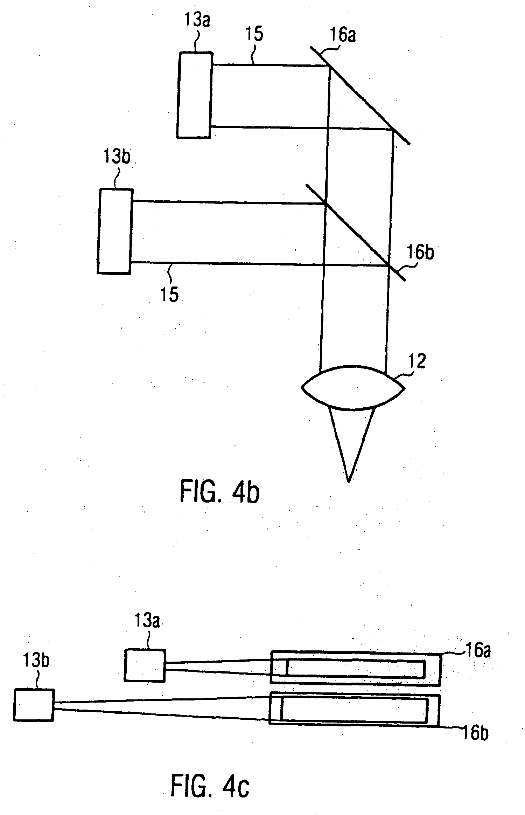

Fig. 4b und 4c zeigen Anordnungen in Drauf- und Vorderansicht, mit der das Licht aus verschiedenen Laserbarren gebündelt werden kann. Zwei Barren 13 a und 13 b sind höhenversetzt angeordnet. Sie geben das Licht 15 auf jeweils einen Spiegel 16a, 16b, die ebenfalls höhenversetzt angeordnet sind. Zwischen den Barren 13a, 13b und den Spiegeln 16a und 16b kann jeweils noch ein Zylinderfinsenarray angeordnet sein, um einen (wenigstens in etwa) kollimierten Strahl 15 zu erhalten. Die von den Spiegeln 16a, 16b reflektierten Strahlen laufen parallel und übereinander, so dass sie von einer einzelnen Einkoppeloptik 12 fokussiert werden können. Der optische Weg zwischen der Einkoppeloptik 12 und den Laserbarren 13a, 13b ist vorteilhafterweise jeweils in etwa gleich lang.

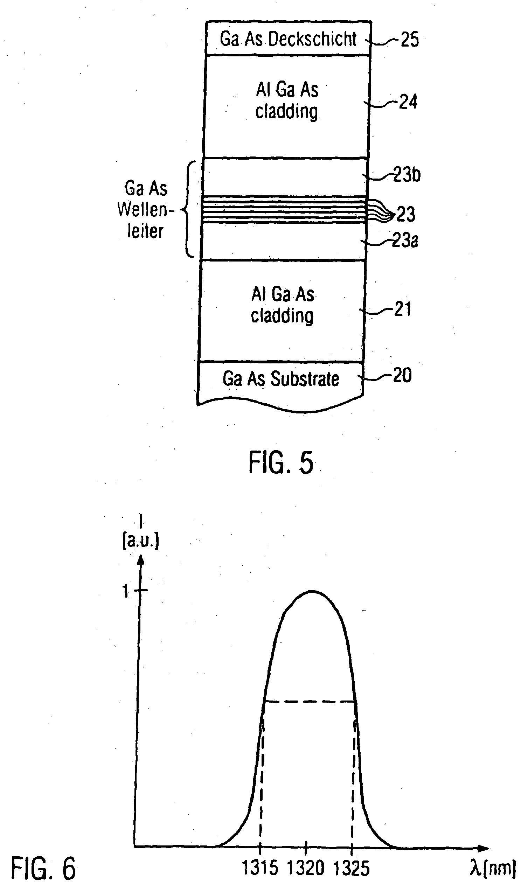

Eine schematische Darstellung der Schichtstruktur des Halbleiterlasers ist in Figur 5 dargestellt. Auf einem Substrat 20 wird eine Claddingschicht 21 aufgewachsen. Auf dieser wird der Wellenleiter ausgebildet. Der Wellenleiter hat einen Brechungsindex, der höher ist, als der der Claddingschicht. Dadurch führt der Wellenleiter die Lichtmode in dem Halbleitermaterial. Innerhalb des Wellenleiters sind verschiedene Schichten 23 angeordnet, die Quantenfilme sein können. In diesen Quantenfilmen 23 können dann auch noch Quantenpunkte angeordnet sein, die die Laserwellenlänge des Lasers festlegen.

Auch ist es möglich nur Quantenpunkte ohne Quantenfilme vorzusehen.

Die Quantenpunkte können auch die Form von Quantendashes haben. Hierbei handelt es sich um Quantenpunkte, die eine länglich Form haben, wie etwa ein lang gezogenes Sechseck oder Ähnliches.

Auf dem Wellenleiter wird eine weitere Claddingschicht 24 aufgebracht und eventuell noch eine Deckschicht 25 angeordnet. Das Substrat 20 und die Claddingschicht 21 weisen vorzugsweise eine gleiche Dotierung auf. Die Claddingschicht 24 und die Deckschicht 25 weisen die entgegengesetzte Dotierung auf. Der Wellenleiter hat beispielsweise eine Breite von 500 nm und kann somit als LOC (Large Optical Cavity) bezeichnet werden. Die Claddingschichten können beispielsweise AlₓGa₁₋ₓAs umfassen bzw. daraus bestehen. Der Aluminiumgehalt und die Dicke dieser Schichten kann geeignet angepasst werden. Die Dicke kann beispielsweise 1,6 Mikrometer sein.

Allgemein können auch δ-dotierte Schichten (δ-doped layers) in dem aktiven Bereich vorgesehen sein, um hohe Laserleistungen zu erreichen.

Der Wellenleiter kann beispielsweise GaAs sein oder umfassen. Ihm kann auch Aluminium, Indium, Phosphor oder Ähnliches hinzugegeben werden. Seine Bandlücke ist kleiner als die der Claddingschicht 21, 24. Das Material des Wellenleiters 23 a, 23 b ist vorzugsweise undotiert.

Werden die Quantenpunkte beispielsweise aus InGaAs gebildet, so können sie auch in InGaAs-Quantenfilmen gebildet werden, sofern der Indiumgehalt in den Punkten wesentlich größer ist.

Die Quantenfilme 23 können auch ohne Quantenpunkte ausgebildet werden. Beispielsweise kann hier GaInAsN als Filmmaterial vorgesehen sein. Diese Materialschichten können zwischen GaAs und/oder GaInAs angeordnet werden. Sowohl die Quantenfilmmaterialien als auch die Barrieren dazwischen können mit Antimon (Sb) oder Phosphor angepasst werden. Dadurch kann die genauer gewünschte Wellenlänge eingestellt werden.

Statt GaInAsN kann auch GaInAsP als Quantenfilm oder Quantenpunktmaterial eingesetzt werden.

Figur 6 zeigt ein typisches Spektrum des durch die Laservorrichtung ausgegebenen Laserlichts. In Figur 6 ist die Intensität in willkürlichen Einheiten über der Wellenlänge in Nanometern aufgetragen. Das dort gezeigte Spektrum hat eine Halbwertsbreite von 10 Nanometern und eine Zentralwellenlänge von 1320 Nanometern.

## Patentansprüche

1. Vorrichtung für die Laserchirurgie mit:
einem Laser (13) und
Mitteln (12, 8) zum Einkoppeln von Laserlicht des Lasers (13) in eine Lichtleitfaser (11, 3)
**dadurch gekennzeichnet, dass**
der Laser (13) ein Festkörperlaser ist, der Laserlicht (15) ausstrahlen kann, das bei der Maximalintensität eine Wellenlänge im Bereich zwischen 1200 und 1330 nm hat,
wobei der Laser mindestens eine Ausgangsleistung von 50 W hat,
und wobei der Festkörperlaser ein Halbleiterlaser ist.

2. Vorrichtung nach Anspruch 1, **dadurch gekennzeichnet, dass** der Wellenlängenbereich der Maximalintensität oberhalb von 1250, 1275, 1300, 1308 oder 1310 nm liegt.

3. Vorrichtung nach Anspruch 1 oder 2, **dadurch gekennzeichnet, dass** der Wellenlängenbereich der Maximalintensität unterhalb von 1325, 1300, 1275, 1250 oder 1225 nm liegt.

4. Vorrichtung nach einem der Ansprüche 1 bis 3, **dadurch gekennzeichnet, dass** die spektrale Halbwertsbreite des Laserlichts mindestens 2, 3, 4, 5, 7, 10, 12 oder 15 nm beträgt.

5. Vorrichtung nach einem der Ansprüche 1 bis 4, **dadurch gekennzeichnet, dass** die spektrale Halbwertsbreite des Laserlichts höchstens 2, 3, 4, 5, 7, 10, 12, 15, 20, 25, 30, 40 nm beträgt.

6. Vorrichtung nach einem der Ansprüche 1 bis 5, **dadurch gekennzeichnet, dass** die Ausgangsleistung des Lasers (13) mindestens 60, 70, 80, 90 oder 100 W beträgt.

7. Vorrichtung nach einem der Ansprüche 1 bis 6, **dadurch gekennzeichnet, dass** der Laser (13) ein Dauerstrichlaser oder ein gepulster Laser (13) ist.

8. Vorrichtung nach einem der Ansprüche 1 bis 7, **dadurch gekennzeichnet, dass** der Laser (13) mehrere Laserelemente (14) umfasst, wie etwa mindestens oder genau 15, 19, 20, 25, 30, 35, 38, 40, 50, 60, 70, 80, 90, 100, 120 oder 150 Laserelemente.

9. Vorrichtung nach Anspruch 8, **dadurch gekennzeichnet, dass** Mittel (12) zum Einkoppeln des Laserlichts der einzelnen Laserelemente (14) in eine gemeinsame Lichtleitfaser (10) vorgesehen sind.

10. Vorrichtung nach einem der Ansprüche 1 bis 9, **dadurch gekennzeichnet, dass** der Laser (13) oder die Laserelemente (14) Quantenpunktlaser und/oder Quantenfilmlaser sind oder umfassen.

11. Vorrichtung nach Anspruch 10, **dadurch gekennzeichnet, dass** die Quantenpunktlaser GaAs und AlGaAs umfassen oder InP und AlGaAsP umfassen.

12. Vorrichtung nach Anspruch 10 oder 11, **dadurch gekennzeichnet, dass** der/die Quantenpunktlaser Quantenpunkte in einem Quantenfilm (23) oder außerhalb von einem Quantenfilm oder in einem Wellenleitermaterial ohne Quantenfilm umfassen.

13. Vorrichtung nach einem der Ansprüche 10 bis 12, **dadurch gekennzeichnet, dass** die Quantenpunkte GaₓIn₁₋ₓAs, GaₓIn₁₋ₓAs_{y}P_{1-y}, GaₓIn₁₋ₓAs_{y}N_{1-y} und/oder GaₓIn₁₋ₓSb umfassen oder daraus bestehen, wobei x und/oder y die Werte von 0 bis 1 annehmen kann.

14. Vorrichtung nach einem der Ansprüche 1 bis 13, **dadurch gekennzeichnet, dass** der Wellenleiter (22) des Halbleiterlasers eine Breite von mindestens 200, 250, 300, 400, 500 oder 600 nm hat.

15. Vorrichtung nach einem der Ansprüche 1 bis 14, **dadurch gekennzeichnet, dass** der Wellenleiter (22) GaₓIn₍₁₋ₓ₎AsᵤPᵥN_{w}Sb_{(1-u-v-w)} umfasst oder daraus besteht, wobei x, u, v und w die Werte 0 bis 1 und alle Zwischenwerte oder Werte aus allen möglichen Zwischenintervallen annehmen kann und u+v+w kleiner oder gleich 1 ist.

16. Vorrichtung nach Anspruch 10, **dadurch gekennzeichnet, dass** der Quantenfilmlaser einen Quantenfilm (23) umfasst, der GaᵤIn₁₋ᵤAsₓN_{y}P_{(1-x-y)}umfasst oder daraus besteht, wobei y die Werte 0 bis 1 oder alle Zwischenwerte oder Werte aus allen möglichen Zwischenintervallen annehmen kann, solange x+y≤1 ist.

17. Vorrichtung nach einem der Ansprüche 1 bis 16, **dadurch gekennzeichnet, dass** eine Kopplung (8) zum lösbaren Anschließen einer Lichtleitfaser (3) vorgesehen ist.

18. Vorrichtung nach einem der Ansprüche 1 bis 17, **dadurch gekennzeichnet, dass** die Vorrichtung eine lösbare Lichtleitfaser (3) umfasst.

19. Vorrichtung nach Anspruch 18, **dadurch gekennzeichnet, dass** die Lichtleitfaser (3) ein Griffteil (4) umfasst.

20. Vorrichtung nach einem der Ansprüche 18 oder 19, **dadurch gekennzeichnet, dass** am distalen Ende der Lichtleitfaser (3) eine Optik (7) vorgesehen ist, mit der ein Lichtfokus in einem Abstand (d) von 1 bis 6 cm, bevorzugterweise 1 bis 5 cm, und noch bevorzugter 1,5 bis 3 cm, von dem Ende der Lichtleitfaser ausgebildet werden kann, wobei die Optik vorzugsweise auswechselbar ist.

21. Vorrichtung nach Anspruch 20, **dadurch gekennzeichnet, dass** die Optik einen Fokus mit einer Halbwertsbreite (b) von 0,2 bis 0,7 mm, vorzugsweise 0,3 bis 0,6 mm und noch bevorzugter 0,4 bis 0,5 mm, erzeugt.

22. Vorrichtung nach einem der Ansprüche 1 bis 21, **dadurch gekennzeichnet, dass** eine Luftkühleinrichtung (5) zum Kühlen des Lasers mit Kühlluft vorgesehen ist.

## Claims

1. A device for laser surgery, comprising:
a laser (13) and
means (12, 8) for coupling laser light of the laser (13) into an optical fiber (11, 3),
**characterized in that**
the laser (13) is a solid-state laser, which is capable of irradiating laser light (15), which at maximum intensity has a wavelength in the range of 1200 to 1330 nm,
wherein the laser has at least an output power of 50 W
and wherein the solid state laser is a semiconductor laser.

2. A device as claimed in claim 1, **characterized in that** the wavelength range of the maximum intensity is above 1250, 1275, 1300, 1308 or 1310 nm.

3. A device as claimed in claim 1 or 2, **characterized in that** the wavelength range of the maximum intensity is below 1325, 1300, 1275, 1250 or 1225 nm.

4. A device as claimed in one of claims 1 to 3, **characterized in that** the spectral full width at half maximum of the laser light is at least 2, 3, 4, 5, 7, 10, 12 or 15 nm.

5. A device as claimed in one of claims 1 to 4, **characterized in that** the spectral full width at half maximum of the laser light is at most 2, 3, 4, 5, 7, 10, 12, 15, 20, 25, 30, 40 nm.

6. A device as claimed in one of claims 1 to 5, **characterized in that** the output power of the laser (13) is at least 60, 70, 80, 90 or 100 W.

7. A device as claimed in one of claims 1 to 6, **characterized in that** the laser (13) is a continuous-wave laser or a pulsed laser (13).

8. A device as claimed in one of claims 1 to 7, **characterized in that** the laser (13) comprises a plurality of laser elements (14), such as at least or exactly 15, 19, 20, 25, 30, 35, 38, 40, 50, 60, 70, 80, 90, 100, 120 or 150 laser elements.

9. A device as claimed in claim 8, **characterized in that** means (12) for coupling laser light of the individual laser elements (14) into a common optical fiber (10) are provided.

10. A device as claimed in one of claims 1 to 9, **characterized in that** the laser (13) or the laser elements (14) are or comprise quantum dot lasers and/or quantum film lasers.

11. A device as claimed in claim 10, **characterized in that** the quantum dot lasers comprise GaAs and AlGaAs or comprise InP and AlGaAsP.

12. A device as claimed in claim 10 or 11, **characterized in that** the quantum dot laser/s comprise/s quantum dots in a quantum film (23) or outside of a quantum film or in an optical waveguide material without a quantum film.

13. A device as claimed in one of claims 10 to 12, **characterized in that** the quantum dots comprise GaₓIn₁₋ₓAs, GaₓIn₁₋ₓAs_{y}P_{1-y}, GaₓIn₁₋ₓAs_{y}N_{1-y} and/or GaₓIn₁₋ₓSb or consist thereof, wherein x and/or y may adopt the values from 0 to 1.

14. A device as claimed in one of claims 1 to 13, **characterized in that** the optical waveguide (22) of the semiconductor laser has a width of at least 200, 250, 300, 400, 500 or 600 nm.

15. A device as claimed in one of claims 1 to 14, **characterized in that** the optical waveguide (22) comprises GaₓIn₍₁₋ₓ₎AsᵤPᵥN_{w}Sb_{(1-u-v-w)} or consists thereof, wherein x, u, v and w may adopt the values 0 to 1 and all intermediate values or values from all possible intermediate intervals and u+v+w is smaller than or equal 1.

16. A device as claimed in claim 10, **characterized in that** the quantum film laser comprises a quantum film (23), which comprises GaᵤIn₁₋ᵤAsₓN_{y}P_{(1-x-y)} or consists thereof, wherein y may adopt the values 0 to 1 or all intermediate values or values from all possible intermediate intervals as long as x+y≤1.

17. A device as claimed in one of claims 1 to 16, **characterized in that** a coupling (8) is provided for the detachable connection of an optical fiber (3).

18. A device as claimed in one of claims 1 to 17, **characterized in that** the device comprises a detachable optical fiber (3).

19. A device as claimed in claim 18, **characterized in that** the optical fiber (3) comprises a handle member (4).

20. A device as claimed in one of claims 18 or 19, **characterized in that** an optical system (7) is provided at the distal end of the optical fiber (3) by means of which a light focus can be formed at a distance (d) of 1 to 6 cm, preferably 1 to 5 cm, even more preferred 1.5 to 3 cm, from the end of the optical fiber, wherein the optical system is preferably exchangeable.

21. A device as claimed in claim 20, **characterized in that** the optical system generates a focus with a full width at half maximum (b) of 0.2 to 0.7 mm, preferably 0.3 to 0.6 mm and even more preferred 0.4 to 0.5 mm.

22. A device as claimed in one of claims 1 to 21, **characterized in that** an air cooling device (5) for cooling the laser with cooling air is provided.

## Revendications

1. Dispositif pour la chirurgie au laser, avec :
un laser (13) et
des moyens (12, 8) pour introduire la lumière laser du laser (13) dans une fibre optique (11, 3),
**caractérisé en ce que** le laser (13) est un laser solide qui peut émettre une lumière laser (15) présentant à une intensité maximale une longueur d'onde dans la plage située entre 1200 et 1330 nm,
le laser ayant au moins une puissance de sortie de 50 W
et le laser solide étant constitué par un laser semiconducteur.

2. Dispositif selon la revendication 1, **caractérisé en ce que** la plage de longueur d'onde de l'intensité maximale est située au-dessus de 1250, 1275, 1300, 1308 ou 1310 nm.

3. Dispositif selon la revendication 1 ou 2, **caractérisé en ce que** la plage de longueur d'onde de l'intensité maximale est située au-dessus de 1325, 1300, 1275, 1250 ou 1225 nm.

4. Dispositif selon l'une des revendications 1 à 3, **caractérisé en ce que** la largeur à mi-hauteur spectrale de la lumière laser est au minimum de 2, 3, 4, 5, 7, 10, 12 ou 15 nm.

5. Dispositif selon l'une des revendications 1 à 4, **caractérisé en ce que** la largeur à mi-hauteur spectrale de la lumière laser est au maximum de 2, 3, 4, 5, 7, 10, 12, 15, 20, 25, 30, 40 nm.

6. Dispositif selon l'une des revendications 1 à 5, **caractérisé en ce que** la puissance de sortie du laser (13) est d'au moins 60, 70, 80, 90 ou 100 W.

7. Dispositif selon l'une des revendications 1 à 6, **caractérisé en ce que** le laser (13) est un laser continu ou un laser pulsé (13).

8. Dispositif selon l'une des revendications 1 à 7, **caractérisé en ce que** le laser (13) comprend plusieurs éléments lasers (14), comme par exemple au moins ou exactement 15, 19, 20, 25, 30, 35, 38, 40, 50, 60, 70, 80, 90, 100, 120 ou 150 éléments lasers.

9. Dispositif selon la revendication 8, **caractérisé en ce qu'**il est prévu des moyens (12) pour introduire la lumière laser des éléments lasers (14) individuels dans une fibre optique commune (10).

10. Dispositif selon l'une des revendications 1 à 9, **caractérisé en ce que** le laser (13) ou les éléments lasers (14) sont ou comprennent des lasers à points quantiques et/ou des lasers à film quantique.

11. Dispositif selon la revendication 10, **caractérisé en ce que** les lasers à points quantiques comprennent GaAs et AlGaAs ou comprennent InP et AlGaAsP.

12. Dispositif selon la revendication 10 ou 11, **caractérisé en ce que** le/les lasers à points quantiques comprennent des points quantiques dans un film quantique (23) ou en dehors d'un film quantique ou dans un matériau guide d'ondes sans film quantique.

13. Dispositif selon l'une des revendications 10 à 12, **caractérisé en ce que** les points quantiques comprennent ou se composent de GaₓIn₁₋ₓAs, GaₓIn₁₋ₓAs_{y}P_{1-y}, GaₓIn₁₋ₓAs_{y}N_{1-y} et/ou GaₓIn₁₋ₓSb, x et/ou y pouvant prendre les valeurs de 0 à 1.

14. Dispositif selon l'une des revendications 1 à 13, **caractérisé en ce que** le guide d'ondes (22) du laser semiconducteur a une largeur d'au moins 200, 250, 300, 400, 500 ou 600 nm.

15. Dispositif selon l'une des revendications 1 à 14, **caractérisé en ce que** le guide d'ondes (22) comprend ou se compose de GaₓIn₍₁₋ₓ₎AsᵤPᵥN_{w}Sb_{(1-u-v-w)}, x, u, v et w pouvant prendre les valeurs 0 à 1 et toutes les valeurs intermédiaires ou des valeurs de tous les intervalles possibles, et u+v+w étant inférieur ou égal à 1.

16. Dispositif selon la revendication 10, **caractérisé en ce que** le laser à film quantique comprend un film quantique (23) qui comprend ou se compose de GaᵤIn₁₋ᵤAsₓN_{y}P_{(1-x-y)}, u, x et y pouvant prendre les valeurs 0 à 1 ou toutes les valeurs intermédiaires ou des valeurs de tous les intervalles possibles, dans la mesure où x+y≤1.

17. Dispositif selon l'une des revendications 1 à 16, **caractérisé en ce qu'**un couplage (8) est prévu pour le raccordement amovible d'une fibre optique (3).

18. Dispositif selon l'une des revendications 1 à 17, **caractérisé en ce que** le dispositif comprend une fibre optique (3) amovible.

19. Dispositif selon la revendication 18, **caractérisé en ce que** la fibre optique (3) comprend un élément formant poignée (4).

20. Dispositif selon l'une des revendications 15 ou 19, **caractérisé en ce qu'**il est prévu à l'extrémité distale de la fibre optique (3) un système optique (7) avec lequel un point focal lumineux peut être formé à une distance (d) de 1 à 6 cm, de préférence 1 à 5 cm et plus spécialement 1,5 à 3 cm de l'extrémité de la fibre optique, le système optique étant de préférence remplaçable.

21. Dispositif selon la revendication 20, **caractérisé en ce que** le système optique produit un point focal avec une largeur à mi-hauteur (b) de 0,2 à 0,7 mm, de préférence 0,3 à 0,6 mm et plus spécialement 0,4 à 0,5 mm.

22. Dispositif selon l'une des revendications 1 à 21, **caractérisé en ce qu'**il est prévu un dispositif de refroidissement à air (5) pour refroidir le laser avec de l'air de refroidissement.
